# EUROPEAN PATENT SPECIFICATION

(11) **EP 0 977 587 B1**
(45) Date of publication and mention of the grant of the patent: **15.06.2005**
(21) Application number: 98910103.5
(22) Date of filing: 02.03.1998
(51) Int. Cl.: A61K 39/12, A61K 39/193, C12N 7/01

(54) **CHIMERIC FLAVIVIRUS VACCINES**
CHIMÄRE IMPFSTOFFE GEGEN FLAVIVIREN
VACCINS CHIMERES A BASE DE FLAVIVIRUS

(30) Priority: 28.02.1997 US 807445; 15.01.1998 US 7664
(43) Date of publication of application: 09.02.2000
(73) Proprietor: Acambis Inc., Cambridge, MA 02139-4169 (US); ST. LOUIS UNIVERSITY, St. Louis, MO 63103 (US)
(72) Inventor: CHAMBERS, Thomas, J., St. Louis, MO 63122 (US); MONATH, Thomas, P., Harvard, MA 01451 (US); GUIRAKHOO, Farshad, Melrose, MA 02176 (US)
(74) Representative: Vossius, Volker, Dr.
(86) International application number: PCT/US1998/003894
(87) International publication number: WO 1998/037911

(56) References cited:
- WO-A-93/06214
- C. RICE ET AL.: "Transcription of infectious yellow fever RNA drom full-length cDNA templates produced by in vitro ligation" THE NEW BIOLOGIST, vol. 1, no. 3, 1989, pages 285-296, XP002107900
- C. DUARTE DOS SANTOS ET AL.: "Complete nucleotide sequence of yellow fever virus vaccine strains 17DD and 17D-213" VIRUS RESEARCH , vol. 35, 1995, pages 35-41, XP002107898
- T. CHAMBERS ET AL.: "Yellow fever/japanese encephalitis chimeric viruses: construction and biological properties" J. VIROL., vol. 73, no. 4, 1999, pages 3095-3101, XP002139409
- BRAY et al., "Construction of Intertypic Chimeric Dengue Viruses by Substitution of Structural Protein Genes", PROC. NATL. ACAD. SCI., USA, November 1991, Vol. 88, pages 10342-10346, XP002910528
- VENUGOPAL et al., "Towards a New Generation of Flavivirus Vaccines", VACCINE, 1994, Vol. 12, No. 11, pages 966-975, XP002910529
- MARCHEVSKY et al., "Phenotypic Analysis of Yellow Fever Virus Derived from Complementary DNA", AMERICAN J. TROPICAL MEDICINE & HYGIENE, 1995, Vol. 52, No. 1, pages 75-80, XP002910530
- PLETNEV A.G. ET AL PROC.NATL.ACAD.SCI.USA vol. 89, 1992, pages 10532 - 10536
- CAUFOUR, P.S. ET AL VIRUS RESEARCH 2001,
- GUIRAKHOO F. ET AL J. VIROL. vol. 74, 2000, pages 5477 - 5485
- ARROYO J. ET AL TRENDS MOL. MED. vol. 7, 2001, pages 350 - 354
- MONATH T.P. ET AL VACCINE vol. 17, 1999, pages 1869 - 1882

## Description

### Background of the Invention

This invention relates to infectious, attenuated viruses useful as vaccines against diseases caused by flaviviruses.

Several members of the flavivirus family pose current or potential threats to global public health. For example, Japanese encephalitis is a significant public health problem involving millions of at risk individuals in the Far East. Dengue virus, with an estimated annual incidence of 100 million cases of primary dengue fever and over 450,000 cases of dengue hemorrhagic fever worldwide, has emerged as the single most important arthropod-transmitted human disease. Other flaviviruses continue to cause endemic diseases of variable nature and have the potential to emerge into new areas as a result of changes in climate, vector populations, and environmental disturbances caused by human activity. These flaviviruses include, for example, St. Louis encephalitis virus, which causes sporadic, but serious acute disease in the midwest, southeast, and western United States; West Nile virus, which causes febrile illness, occasionally complicated by acute encephalitis, and is widely distributed throughout Africa, the Middle East, the former Soviet Union, and parts of Europe; Murray Valley encephalitis virus, which causes endemic nervous system disease in Australia; and Tick-borne encephalitis virus, which is distributed throughout the former Soviet Union and eastern Europe, where its ixodid tick vector is prevalent and responsible for a serious form of encephalitis in those regions.

Hepatitis C virus (HCV) is another member of the flavivirus family, with a genome organization and replication strategy that are similar, but not identical, to those of the flaviviruses mentioned above. HCV is transmitted mostly by parenteral exposure, is associated with chronic hepatitis that can progress to cirrhosis and hepatocellular carcinoma, and is a leading cause of liver disease requiring orthotopic transplantation in the United States.

The Flaviviridae family is distinct from the alphaviruses (*e.g*., WEE, VEE, EEE, SFV, etc.) and currently contains three genera, the flaviviruses, the pestiviruses, and the hepatitis C viruses. Fully processed mature virions of flaviviruses contain three-structural proteins, envelope (E), capsid (C), and membrane (M), and seven non-structural proteins (NS1, NS2A, NS2B, NS3, NS4A, NS4B, and NS5). Immature flavivirions found in infected cells contain pre-membrane (prM) protein, which is the precursor to the M protein.

After binding of virions to host cell receptors, the E protein undergoes an irreversible conformational change upon exposure to the acidic pH of endosomes, causing fusion between the envelope bilayers of the virions and endocytic vesicles, thus releasing the viral genome into the host cytosol. PrM-containing tick-borne encephalitis (TBE) viruses are fusion-incompetent, indicating that proteolytic processing of prM is necessary for the generation of fusion-competent and fully infectious virions (Guirakhoo *et al*., J. Gen. Virol. 72(Pt. 2):333-338, 1991). Using ammonium chloride late in virus replication cycle, prM-containing Murray Valley encephalitis (MVE) viruses were produced and shown to be fusion incompetent. By using sequence-specific peptides and monoclonal antibodies, it was demonstrated that prM interacts with amino acids 200-327 of the E protein. This interaction is necessary to protect the E protein from the irreversible conformational changes caused by maturation in the acidic vesicles of the exocytic pathway (Guirakhoo *et al*., Virology 191:921-931, 1992).

The cleavage of prM to M protein occurs shortly before release of virions by a furin-like cellular protease (Stadler *et al*., J. Virol. 71:8475-8481, 1997), which is necessary to activate hemagglutinating activity, fusogenic activity, and infectivity of virions. The M protein is cleaved from its precursor protein (prM) after the consensus sequence R-X-R/K-R (X is variable), and incorporated into the virus lipid envelope together with the E protein.

Cleavage sequences have been conserved not only within flaviviruses, but also within proteins of other, unrelated viruses, such as PE2 of murine coronaviruses, PE2 of alphaviruses, HA of influenza viruses, and p 160 of retroviruses. Cleavage of the precursor protein is essential for virus infectivity, but not particle formation. It was shown that, in case of a TBE-dengue 4 chimera, a change in the prM cleavage site resulted in decreased neurovirulence of this chimera (Pletnev *et al*., J. Virol. 67:4956-4963, 1993), consistent with the previous observation that efficient processing of the prM is necessary for full infectivity (Guirakhoo *et al*., 1991, *supra*, 1992, *supra*; Heinz *et al*., Virology 198:109-117, 1994). Antibodies to prM protein can mediate protective immunity, apparently due to neutralization of released virions that contain some uncleaved prM. The proteolytic cleavage site of the PE2 of VEE (4 amino acids) was deleted by site-directed mutagenesis of the infectious clone (Smith *et al*., ASTMH meeting, December 7-11, 1997). Deletion mutants replicated with high efficiency and PE2 proteins were incorporated into particles. This mutant was evaluated in non-human primates and shown to cause 100% seroconversion and protected all immunized monkeys from a lethal challenge.

### Summary of the Invention

The invention features chimeric, live, infectious, attenuated viruses that are each composed of:
(a) a first yellow fever virus (*e*.*g*., strain 17D), representing a live, attenuated vaccine virus, in which the nucleotide sequence for a prM-E protein is either deleted, truncated, or mutated so that the functional prM-E protein of the first flavivirus is not expressed, and
(b) integrated into the genome of the first flavivirus, a nucleotide sequence encoding the viral envelope (prM-E proteins) of a second, different flavivirus, so that the prM-E protein of the second flavivirus is expressed from the altered genome of the first flavivirus, wherein the signal sequence at the C/prM junction of the yellow fever virus is maintained.

The chimeric virus is thus composed of the genes and gene products responsible for intracellular replication belonging to the first flavivirus and the genes and gene products of the envelope of the second flavivirus. Since the viral envelope contains all of the antigenic determinants responsible for inducing neutralizing antibodies, the result of infection with the chimeric virus is that such antibodies are generated only against the second flavivirus.

A preferred live virus for use as the first flavivirus in the chimeric viruses of the invention is the yellow fever virus. At least one vaccine has already employed this live, attenuated virus; the vaccine is known as YF17D and has been used for human immunization for over 50 years. YF17D vaccine is described in a number of publications, including publications by Smithburn *et al.* ("Yellow Fever Vaccination," World Health Org., p. 238,1956), and Freestone (in Plotkin *et al*., (Eds.), Vaccines, 2^{nd} edition, W.B. Saunders, Philadelphia, 1995). In addition, the yellow fever virus has been studied at the genetic level (Rice *et al*., Science 229:726-733, 1985), and information correlating genotype and phenotype has been established (Marchevsky *et al*., Am. J. Trop. Med. Hyg. 52:75-80, 1995).

Preferred flaviviruses for use as the second flavivirus in the chimeric viruses of the invention, and thus sources of immunizing antigen, include Japanese Encephalitis (JE), Dengue (DEN, *e*.*g*., any of Dengue types 1-4), Murray Valley Encephalitis (MVE), St. Louis Encephalitis (SLE), West Nile (WN), Tick-borne Encephalitis (TBE), and Hepatitis C (HCV) viruses. Additional flaviviruses for use as the second flavivirus include Kunjin virus, Central European Encephalitis virus, Russian Spring-Summer Encephalitis virus, Powassan virus, Kyasanur Forest Disease virus, and Omsk Hemorrhagic Fever virus. In a preferred chimeric virus of the invention, the prM-E protein coding sequence of the second flavivirus is substituted into the prM-E protein coding sequence of the live yellow fever virus. In a preferred chimeric virus, the prM-E protein coding sequence is derived from an attenuated virus strain, such as a vaccine strain. Also, as is described further below, the prM portion of the protein can contain a mutation that prevents cleavage to generate mature membrane protein.

Also included in the invention is the use of the chimeric flaviviruses of the invention in the preparation of medicaments for preventing or treating flavivirus infection; nucleic acid molecules encoding the chimeric flaviviruses of the invention; and methods of manufacturing the chimeric flaviviruses of the invention.

The invention provides several advantages. For example, because they are live and replicating, the chimeric viruses of the invention can be used to produce long-lasting protective immunity. Because the viruses have the replication genes of an attenuated virus (*e*.*g*., Yellow Fever 17D), the resulting chimeric virus is attenuated to a degree that renders it safe for use in humans.

Other features and advantages of the invention will be apparent from the following detailed description, the drawings, and the claims.

### Brief Description of the Drawings

Fig. 1 is a schematic representation of genetic manipulation steps that were carried out in order to construct a Yellow-Fever/Japanese Encephalitis (YF/JE) chimeric virus of the invention.
Fig. 2 is a set of growth curves for chimeric YF/JE viruses of the invention in cell cultures acceptable for preparation of a human vaccine.
Fig. 3 is a graph showing a growth comparison between RMS (Research Master Seed, YF/JE SA₁₄-14-2) and YF-Vax in MRC-5 cells.
Fig. 4 is a graph and a table showing the results of a mouse neurovirulence analysis carried out with a YF/JE chimeric virus of the invention.
Fig. 5 is a schematic representation of a two plasmid system for generating chimeric YF/DEN-2 virus. The strategy is essentially as described for the YF/JE chimeric virus.

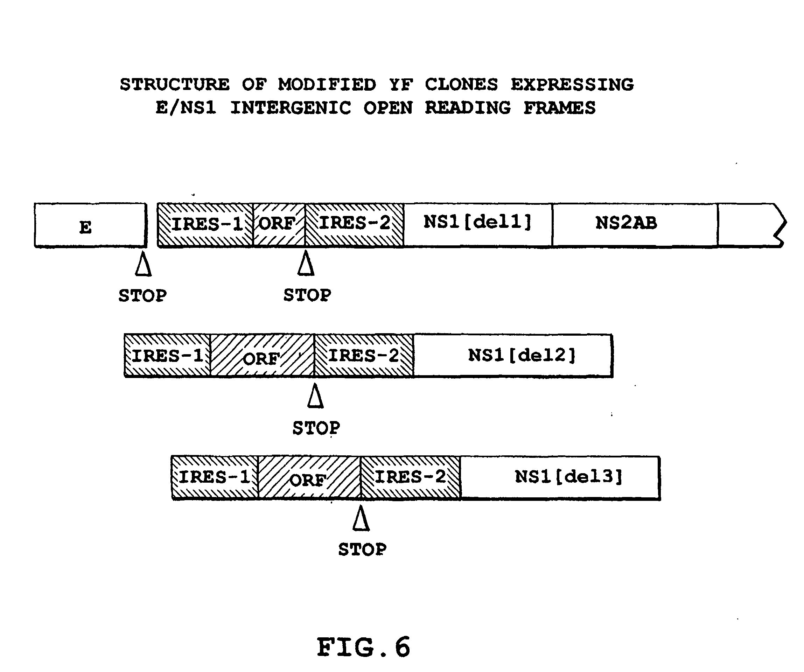

Fig. 6 is a schematic representation of the structure of modified YF clones designed to delete portions of the NS1 protein and/or express foreign proteins under control of an internal ribosome entry site (IRES). The figure shows only the E/NS1 region of the viral genome. A translational stop codon is introduced at the carboxyl terminus of the envelope (E) protein. Downstream translation is initiated within an intergenic open reading frame (ORF) by IRES-1, driving expression of foreign proteins (*e.g*., HCV proteins E1 and/or E2). The second IRES (IRES-2) controls translational initiation of the YF nonstructural region, in which nested, truncated NS1 proteins (*e.g.*, NS1del-1, NS1del-2, or NS1del-3) are expressed. The size of the NS1 deletion is inversely proportional to that of the ORF linked to IRES-1.
Fig. 7 is a graph showing the neutralizing antibody response of mice immunized with a YF/JE SA₁₄-14-2 chimeric vaccine.

### Detailed Description

The invention provides chimeric flaviviruses that can be used in vaccination methods against flavivirus infection. Construction and analysis of chimeric flaviviruses of the invention, such as chimeras of yellow fever virus and Japanese Encephalitis (JE), Dengue types 1-4 (DEN 1-4), Murray Valley Encephalitis (MVE), St Louis Encephalitis (SLE), West Nile (WN), Tick-borne Encephalitis (TBE), and Hepatitis C (HCV) viruses are described as follows.

Flavivirus proteins are produced by translation of a single, long open reading frame (encoding, *i*.*a*., the structural proteins, capsid (C), pre-membrane (pr-M), and envelope (E), as well as non-structural proteins) and a complex series of post-translational proteolytic cleavages. The chimeric flaviviruses of the invention, as is discussed above, include those in which the pr-M and E - proteins of one flavivirus have been replaced by the pr-M and E proteins of another flavivirus. Thus, creation of these chimeric flaviviruses involves the generation of novel junctions between the capsid and pre-membrane proteins, and the envelope protein and the non-structural region (NS1), of two different flaviviruses. Cleavage between each of these sets of proteins (C and pr-M, and E and NS1) occurs during the natural proteolytic processing of flavivirus proteins, and requires the presence of signal sequences flanking the junctions of the cleavage sites.

In the chimeric flaviviruses of the invention, it is preferred that the signal sequences of the yellow fever viruses making up the chimeras are maintained, so that proper cleavage between the C and pr-M and E and NS1 proteins can efficiently take place. A signal sequence can include as its last residue an amino acid with a small, uncharged side chain, such as alanine, glycine, serine, cysteine, threonine, or glutamine.

### Construction of cDNA Templates for Generation of YF/JE Chimeric Virus

The derivation of full-length cDNA templates for YF/JE chimeras of the invention described below employed a strategy similar to that earlier workers used to regenerate YF17D from cDNA for molecular genetic analysis of YF replication. The strategy is described, *e*.*g*., by Nestorowicz *et al*. (Virology 199:114-123, 1994).

Briefly, derivation of a YF/JE chimera of the invention involves the following. YF genomic sequences are propagated in two plasmids (YF5'3'IV and YFM5.2), which encode the YF sequences from nucleotides 1-2,276 and 8,279-10,861 (YF5'3'IV) and from 1,373-8,704 (YFM5.2)(Rice *et al*., The New Biologist 1:285-296, 1989). Full-length cDNA templates are generated by ligation of appropriate restriction fragments derived from these plasmids. This method has been the most reliable for ensuring stable expression of YF sequences and generation of RNA transcripts of high specific infectivity.

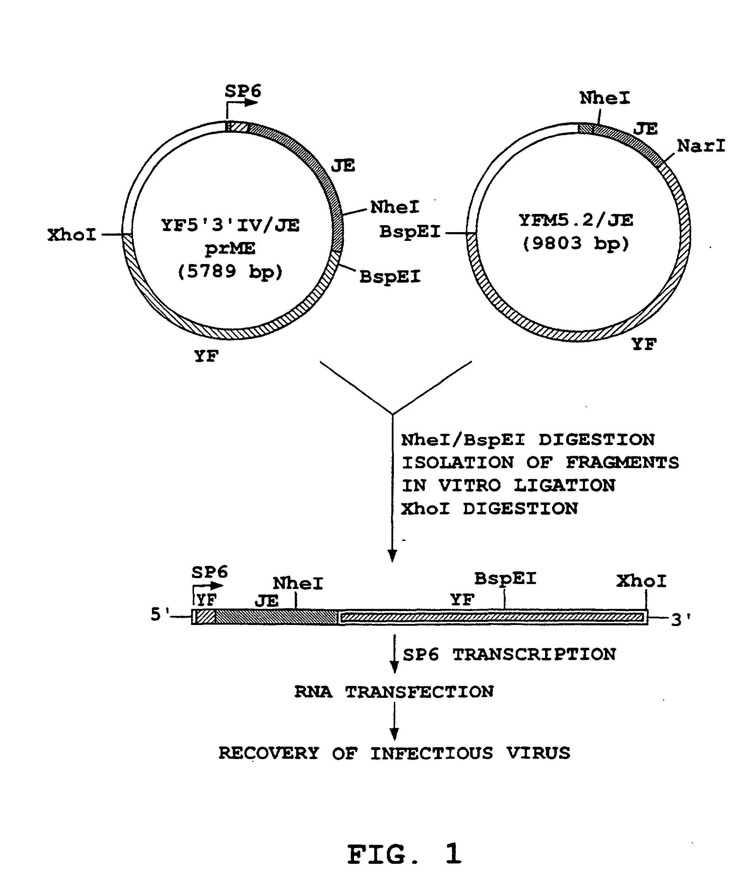

Our strategy for construction of chimeras involves replacement of YF sequences within the YF5'3TV and YFM5.2 plasmids by the corresponding JE sequences from the start of the prM protein (nucleotide 478, amino acid 128) through the E/NS1 cleavage site (nucleotide 2,452, amino acid 817). In addition to cloning of JE cDNA, several steps were required to introduce or eliminate restriction sites in both the YF and JE sequences to permit *in vitro* ligation. The structure of the template for regenerating chimeric YF (C)/JE (prM-E) virus is shown in Fig. 1. A second chimera, encoding the entire JE structural region (C-prM-E) was engineered using a similar strategy.

### Molecular Cloning of the JE Virus Structural Region

Clones of authentic JE structural protein genes were generated from the JE SA₁₄-14-2 strain (JE live, attenuated vaccine strain), because the biological properties and molecular characterization of this strain are well-documented (see, *e*.*g*., Eckels *et al*., Vaccine 6:513-518, 1988; JE SA₁₄-14-2 virus is available from the Centers for Disease Control, Fort Collins, Colorado and the Yale Arbovirus Research Unit, Yale University, New Haven, Connecticut, which are World Health Organization-designated Reference Centers for Arboviruses in the United States). JE SA₁₄-14-2 virus at passage level PDK-5 was obtained and passaged in LLC-MK₂ cells to obtain sufficient amounts of virus for cDNA cloning. The strategy we used involved cloning the structural region in two pieces that overlap at an *Nhe*I site (JE nucleotide 1,125), which can then be used for *in vitro* ligation.

RNA was extracted from monolayers of infected LLC-MK₂ cells and first strand synthesis of negative sense cDNA was carried out using reverse transcriptase with a negative sense primer (JE nucleotide sequence 2,456-71) containing nested *Xba*I and *Nar*I restriction sites for cloning initially into pBluescript II KS(+), and subsequently into YFM5.2(*Nar*I), respectively. First strand cDNA synthesis was followed by PCR amplification of the JE sequence from nucleotides 1,108-2,471 using the same negative sense primer and a positive sense primer (JE nucleotides sequence 1,108-1,130) containing nested *Xba*I and *Nsi*I restriction sites for cloning into pBluescript and YFM5.2(*Nar*I), respectively. JE sequences were verified by restriction enzyme digestion and nucleotide sequencing. The JE nucleotide sequence from nucleotides 1 to 1,130 was derived by PCR amplification of negative strand JE cDNA using a negative sense primer corresponding to JE nucleotides 1,116 to 1,130 and a positive sense primer corresponding to JE nucleotides 1 to 18, both containing an *EcoR*I restriction site. PCR fragments were cloned into pBluescript and JE sequences were verified by nucleotide sequencing. Together, this represents cloning of the JE sequence from nucleotides 1-2,471 (amino acids 1-792).

### Construction of YF5'3'IV/JE and YFM5.2/JE Derivatives

To insert the C terminus of the JE envelope protein at the YF E/NS1 cleavage site, a unique *Nar*I restriction site was introduced into the YFM5.2 plasmid by oligonucleotide-directed mutagenesis of the signalase sequence at the E/NS1 cleavage site (YF nucleotides 2,447-2,452, amino acids 816-817) to create YFM5.2(*Nar*I). Transcripts derived from templates incorporating this change were checked for infectivity and yielded a specific infectivity similar to the parental templates (approximately 100 plaque-forming units/250 nanograms of transcript). The JE sequence from nucleotides 1,108 to 2,471 was subcloned from several independent PCR-derived clones of pBluescript/JE into YFM5.2(*Nar*I) using the unique *Nsi*I and *Nar*I restriction sites. YF5'3'IV/JE clones containing the YF 5' untranslated region (nucleotides 1-118) adjacent to the JE prM-E region were derived by PCR amplification.

To derive sequences containing the junction of the YF capsid and JE prM, a negative sense chimeric primer spanning this region was used with a positive sense primer corresponding to YF5'3'IV nucleotides 6,625-6,639 to generate PCR fragments that were then used as negative sense PCR primers in conjunction with a positive sense primer complementary to the pBluescript vector sequence upstream of the *EcoR*I site, to amplify the JE sequence (encoded in reverse orientation in the pBluescript vector) from nucleotide 477 (N-terminus of the prM protein) through the *Nhe*I site at nucleotide 1,125. The resulting PCR fragments were inserted into the YF5'3'IV plasmid using the *Not*I and *EcoR*I restriction sites. This construct contains the SP6 promoter preceding the YF 5'-untranslated region, followed by the sequence: YF (C) JE (prM-E), and contains the *Nhe*I site (JE nucleotide 1,125) required *for in vitro* ligation.

### Engineering YFM5.2 and YF5'3 'IV to Contain Restriction Sites for in vitro Ligation

In order to use the *Nhe*I site within the JE envelope sequence as a 5' *in vitro* ligation site, a redundant *Nhe*I site in the YFM5.2 plasmid (nucleotide 5,459) was eliminated. This was accomplished by silent mutation of the YF sequence at nucleotide 5,461 (T→C; alanine, amino acid 1820). This site was incorporated into YFM5.2 by ligation of appropriate restriction fragments and introduced into YFM5.2(*Nar*I)/JE by exchange of an *Nsi*I/*Nar*I fragment encoding the chimeric YF/JE sequence.

To create a unique 3' restriction site for *in vitro* ligation, a *BspE*I site was engineered downstream of the *Aat*II site normally used to generate full-length templates from YF5'3'IV and YFM5.2. (Multiple *Aat*II sites are present in the JE structural sequence, precluding use of this site for *in vitro* ligation.) The *BspE*I site was created by silent mutation of YF nucleotide 8,581 (A→C; serine, amino acid 2,860) and was introduced into YFM5.2 by exchange of appropriate restriction fragments. The unique site was incorporated into YFM5.2/JE by exchange of the *Xba*I/*Sph*I fragment and into the YF5'3'IV/JE(prM-E) plasmids by three-piece ligation of appropriate restriction fragments from these parent plasmids and from a derivative of YFM5.2 (*BspE*I) deleting the YF sequence between the *EcoR*I sites at nucleotides 1 and 6,912.

### Exchange of JE Nakayama cDNA into YF/JE Chimeric Plasmids

Because of uncertainty about the capacity of the PCR-derived JE SA₁₄₋14-2 structural region to function properly in the context of the chimeric virus, we used cDNA from a clone of the JE Nakayama strain that has been extensively characterized in expression experiments and for its capacity to induce protective immunity (see, *e.g.*, McIda *et al*., Virology 158:348-360, 1987; the JE Nakayama strain is available from the Centers for Disease Control, Fort Collins, Colorado, and the Yale Arbovirus Research Unit, Yale University, New Haven, Connecticut). The Nakayama cDNA was inserted into the YF/JE chimeric plasmids using available restriction sites (*Hind*III to *Pvu*II and *Bpm*I to *Mun*I) to replace the entire prM-E region in the two plasmid system except for a single amino acid, serine, at position 49, which was left intact in order to utilize the *Nhe*I site for *in vitro* ligation. The entire JE region in the Nakayama clone was sequenced to verify that the replaced cDNA was authentic (Table 2).

### Generation of Full-Length cDNA Templates, RNA Transfection, and Recovery of Infectious Virus

Procedures for generating full-length cDNA templates are essentially as described in Rice *et al*. (The New Biologist 1:285-96, 1989) (see Fig. 1). In the case of chimeric templates, the plasmids YF5'3'IV/JE(prM-E) and YFM5.2/JE are digested with *Nhe*I/*BspE*I and *in vitro* ligation is performed using 50 nanograms of purified fragments in the presence of T4 DNA ligase. The ligation products are linearized with *Xho*I to allow run-off transcription. SP6 transcripts are synthesized using 50 nanograms of purified template, quantitated by incorporation of ³H-UTP, and integrity of the RNA is verified by non-denaturing agarose gel electrophoresis. Yields range from 5 to 10 micrograms of RNA per reaction using this procedure, most of which is present as full-length transcripts. Transfection of RNA transcripts in the presence of cationic liposomes is carried out as described by Rice *et al*. (*supra*) for YF17D. In initial experiments, LLC-MK₂ cells were used for transfection and quantitation of virus, since we have determined the permissiveness for replication and plaque formation of the parental strains of YF and JE. Table 1 illustrates typical results of transfection experiments using Lipofectin (GIBCO/BRL) as a transfection vehicle. Vero cell lines have also been used routinely for preparation of infectious virus stocks, characterization of labeled proteins, and neutralization tests.

### Nucleotide Sequencing of Chimeric cDNA Templates

Plasmids containing the chimeric YF/JE cDNA were subjected to sequence analysis of the JE portion of the clones to identify the correct sequences of the SA₁₄-14-2 and Nakayama envelope protein. The nucleotide sequence differences between these constructs in comparison to the reported sequences (McAda *et al*., *supra*) are shown in Table 2.

### Structural and Biological Characterization of Chimeric YF/JE Viruses

The genomic structure of chimeric YF/JE viruses recovered from transfection experiments was verified by RT/PCR-based analysis of viral RNA harvested from infected cell monolayers. These experiments are performed in order to eliminate the possibility that virus stocks are contaminated during transfection procedures. For these experiments, first-pass virus was used to initiate a cycle of infection, in order to eliminate any possible artifacts generated by the presence of residual transfected viral RNA. Total RNA extracts of cells infected with either the YF/JE (prM-E)-SA₁₄-14-2 or YF/JE (prM-E)-Nakayama chimera were subjected to RT/PCR using YF and JE-specific primers that allowed recovery of the entire structural region as two PCR products of approximately 1 kilobase in size. These products were then analyzed by restriction enzyme digestion using the predicted sites within the JE SA₁₄-14-2 and Nakayama sequences that allow differentiation of these viruses. Using this approach, the viral RNA was demonstrated to be chimeric and the recovered viruses were verified to have the appropriate restriction sites. The actual C-prM boundary was then verified to be intact at the sequence level by cycle sequence analysis across the chimeric YF/JE C-prM junction.

The presence of the JE envelope protein in the two chimeras was verified by both immunoprecipitation with JE-specific antisera and by plaque reduction neutralization testing using YF and JE-specific antisera. Immunoprecipitation of ³⁵S-labeled extracts of LLC-MK₂ cells infected with the chimeras using a monoclonal antibody to the JE E protein showed that the JE envelope protein could be recovered as a 55 kD protein, whereas the same antisera failed to immunoprecipitate a protein from YF-infected cells. Both JE and YF hyperimmune sera demonstrated cross-reactivity for the two envelope proteins, but the size difference between the proteins (YF=53 kD, unglycosylated; JE=55 kD, glycosylated) could reproducibly be observed. Use of YF monoclonal antibodies was not satisfactory under the immunoprecipitation conditions, thus, the specificity was dependent on the JE monoclonal antibodies in this analysis. Plaque reduction neutralization testing (PRNT) was performed on the chimeric viruses and the YF and JE SA₁₄-14-2 viruses using YF and JE-specific hyperimmune ascitic fluid (ATCC) and YF-specific purified IgG (monoclonal antibody 2E10). Significant differences in the 50% plaque reduction titer of these antisera were observed for the chimeras when compared to the control viruses in these experiments (Table 3). Thus, epitopes required for neutralization are expressed in the infectious chimeric YF/JE viruses.

### Growth Properties in Cell Culture

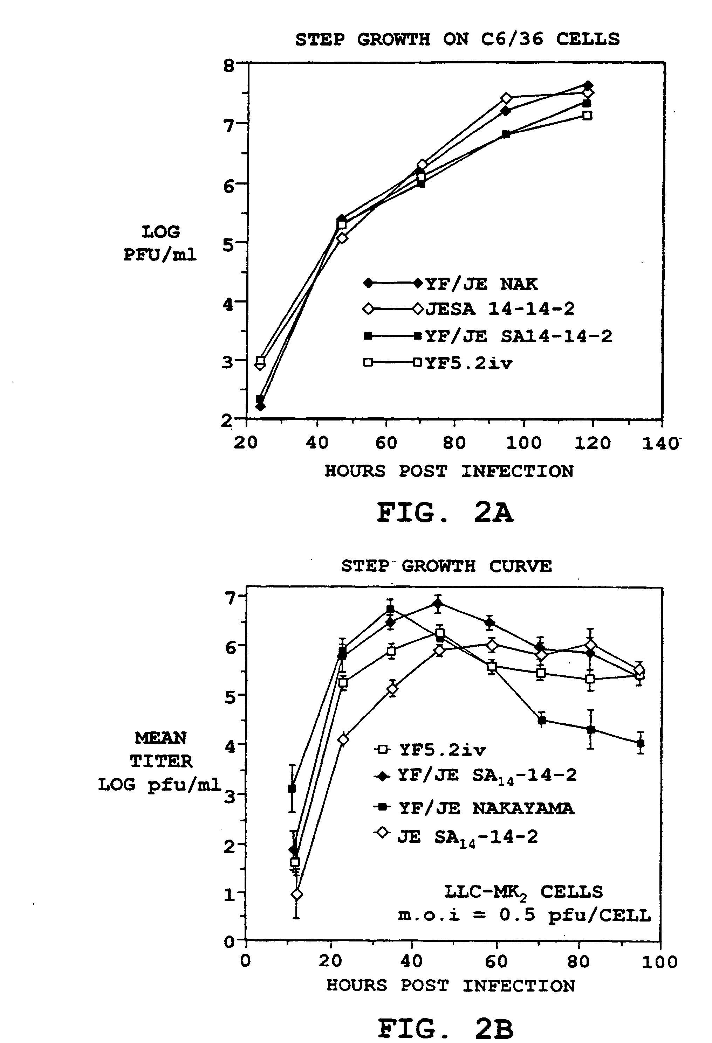

The growth capacity of the chimeras has been examined quantitatively in cell lines of both primate and mosquito origin. Fig. 2 illustrates the cumulative growth curves of the chimeras on LLC-MK₂ cells after low multiplicity infection (0.5 plaque-forming units/cell). In this experiment, YF5.2iv (cloned derivative) and JE SA₁₄-14-2 (uncloned) viruses were used for comparison. Both chimeric viruses reached a maximal virus yield of approximately one log higher than either parental virus. In the case of the YF/JE SA₁₄-14-2 chimera, the peak of virus production occurred 12 hours later than the YF/JE Nakayama chimera (50 hours vs. 38 hours). The YF/JE Nakayama chimera exhibited considerably more cytopathic effects than the YF/JE SA₁₄-14-2 chimera on this cell line. A similar experiment was carried out in C6/36 cells after low multiplicity infection (0.5 plaque-forming units/cell). Fig. 2 also illustrates the growth kinetics of the viruses in this invertebrate cell line. Similar virus yields were obtained at all points used for virus harvest in this experiment, further substantiating the notion that chimeric viruses are not impaired in replication efficiency.

### Comparison of Growth Kinetics of the RMS (YF/JE SA₁₄-14-2) with YF 17D Vaccine in MRC-5 Cells

An experiment was performed to assess the ability of the vaccine candidate to propagate in a cell line acceptable for human vaccines. Commercial Yellow Fever 17D vaccine (YF-Vax®) was obtained from Connaught Laboratories, Swiftwater, PA. MRC-5 (diploid human embryonal lung cells) were purchased from ATCC (171-CCL, Batch#: F-14308, passage 18) and grown in EMEM, 2 mM L-Gln, Earle's BSS adjusted to contain 1.5 g/L sodium bicarbonate, 0.1 mM non-essential amino acids, and 10% FBS.

To compare growth kinetics of RMS (Research Master Seed, YF/JE SA₁₄-14-2) with YF-Vax®, cells were grown to 90% confluency and infected with RMS or YF-Vax® at an MOI of 0.1 pfu. Since MRC-5 cells generally grow slowly, these cells were kept for 10 days post inoculation. Samples were frozen daily for 7-10 days and infectivity determined by plaque assay in Vero cells.

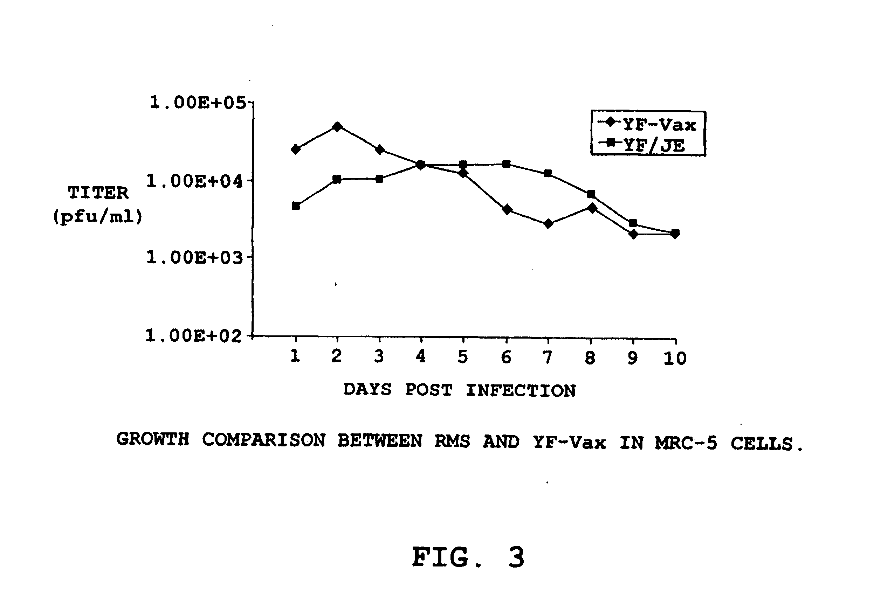

YF-Vax® and the YF/JE chimera grew to modest titers in MRC-5 cells (Fig. 3). The peak titer was ∼4.7 log₁₀ pfu for YF-Vax® achieved on the second day and was slightly lower, 4.5 log₁₀ pfu, for the RMS after 6 days.

### Neurovirulence Testing in Normal Adult Mice

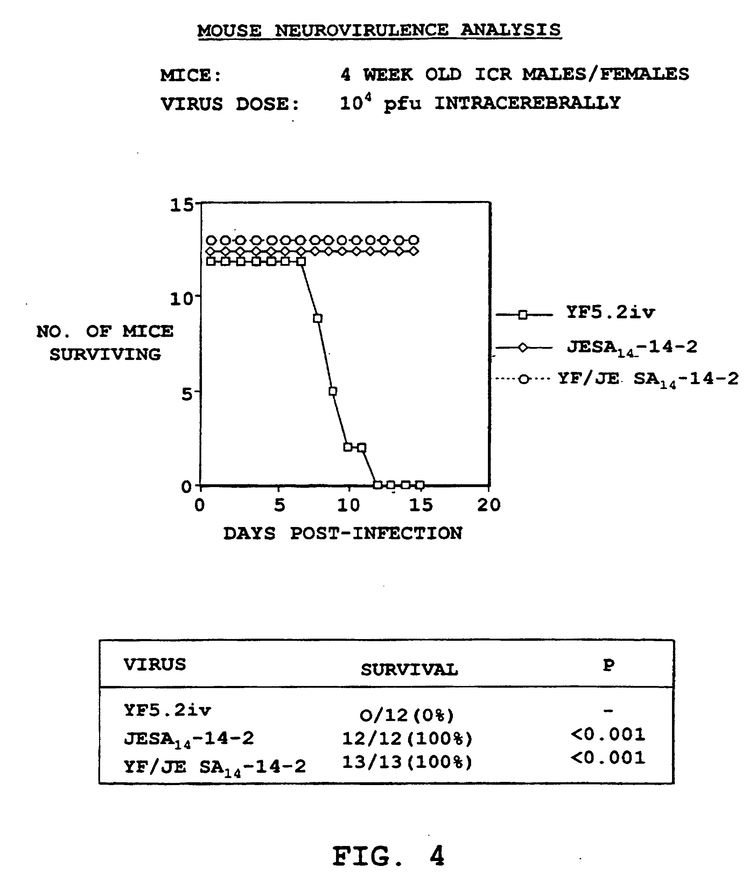

The virulence properties of the YF/JE SA₁₄-14-2 chimera was analyzed in young adult mice by intracerebral inoculation. Groups of 10 mice (4 week old male and female ICR mice, 5 each per group) were inoculated with 10,000 plaque-forming units of the YF/JE SA₁₄-14-2 chimera, YF5.2iv, or JE SA₁₄-14-2 and observed daily for 3 weeks. The results of these experiments are illustrated in Fig. 4. Mice receiving the YF5.2iv parent succumbed by approximately one week post-inoculation. No mortality or illness was observed among mice receiving either the JE SA₁₄-14-2 parent or the chimera. The inocula used for the experiments were titered at the time of injection and a subgroup of the surviving mice were tested for the presence of neutralizing antibodies to confirm that infection had taken place. Among those tested, titers against the JE SA₁₄-14-2 virus were similar for animals receiving either this strain or the chimera.

The results of additional experiments investigating the neurovirulence of the YF/JE SA₁₄-14-2 chimera in mice are illustrated in Table 4. In these experiments, all of the mice inoculated with YF5.2iv died within 7-8 days. In contrast, none of the mice inoculated with YF/JE SA₁₄-14-2 died during two weeks of post-inoculation observation.

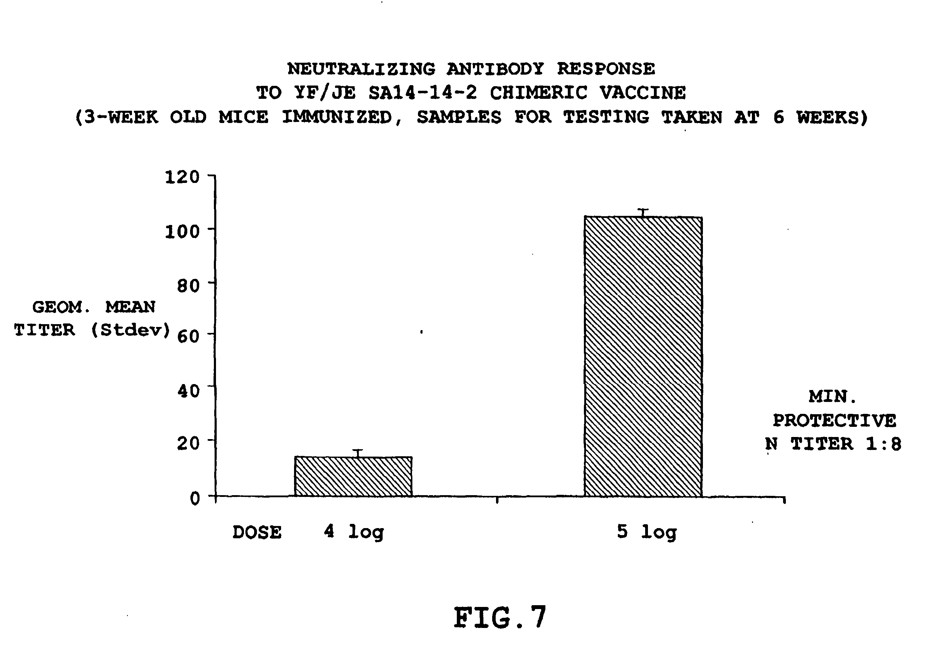

The results of experiments investigating the neuroinvasiveness and pathogenesis of YF/JE chimeras are illustrated in Table 5. In these experiments, the chimeric viruses were inoculated into 3 week old mice at doses varying between 10,000 and 1 million plaque-forming units via the intraperitoneal route. None of the mice inoculated with YF/JE Nakayama or YF/JE SA₁₄-14-2 died during three weeks of post-inoculation observation, indicating that the virus was incapable of causing illness after peripheral inoculation. Mice inoculated with YF/JE SA₁₄-14-2 developed neutralizing antibodies against JE virus (Fig. 7).

### Construction of cDNA Templates for Generation of Yellow Fever/Dengue (YF/DEN) Chimeric Viruses

Derivation of chimeric Yellow Fever/Dengue (YF/DEN) viruses is described as follows, which, in principle, is carried out the same as construction of the YF/JE chimera described above. Other flavivirus chimeras can be engineered with a similar strategy, using natural or engineered restriction sites and, for example, oligonucleotide primers as shown in Table 6.

### Construction of YF/DEN Chimeric Virus

Although several molecular clones for dengue viruses have been developed, problems have commonly been encountered with stability of viral cDNA in plasmid systems, and with the efficiency of replication of the recovered virus. We chose to use a clone of DEN-2 developed by Dr. Peter Wright, Dept. of Microbiology, Monash University, Clayton, Australia, because this system is relatively efficient for regenerating virus and employs a two-plasmid system similar to our own methodology. The complete sequence of this DEN-2 clone is available and facilitated the construction of chimeric YF/DEN templates because only a few modifications of the YF clone were required. The relevant steps are outlined as follows.

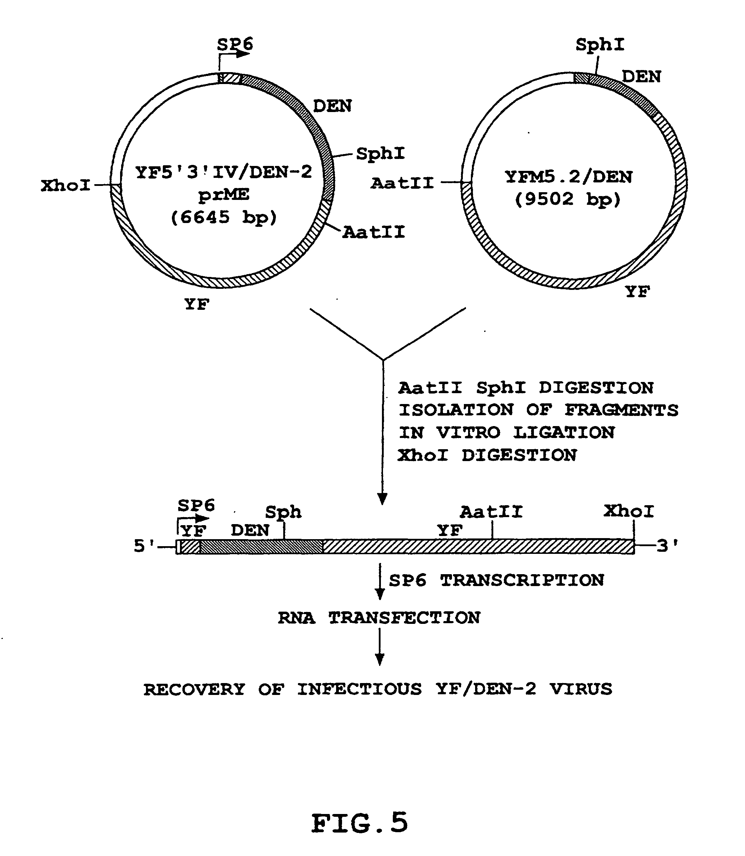

Similar to the two plasmid system for YF5.2iv and YF/JE viruses, the YF/DEN system uses a unique restriction site within the DEN-2 envelope protein (E) as a breakpoint for propagating the structural region (prM-E) within the two plasmids, hereinafter referred to as YFS'3'IV/DEN (prM-E') and YFM5.2/DEN (E'-E) (see Fig. 5). The two restriction sites for *in vitro* ligation of the chimeric template are *Aat*II and *Sph*I. The recipient plasmid for the 3' portion of the DEN E protein sequence is YFM5.2(*Nar*I[+]*Sph*I[-]). This plasmid contains the *Nar*I site at the E/NSI junction, which was used for insertion of the carboxyl terminus of the JE E protein. It was further modified by elimination of an extra *Sph*I site in the NS5 protein region by silent site-directed mutagenesis. This allowed insertion of DEN-2 sequence from the unique *Sph*I site to the *Nar*I site by simple directional cloning. The appropriate fragment of DEN-2 cDNA was derived by PCR from the DEN-2 clone MON310 furnished by Dr. Wright. PCR primers included a 5' primer flanking the *Sph*I site and a 3' primer homologous to the DEN-2 nucleotides immediately upstream of the signalase site at the E/NSI junction and replacing the signalase site by substitutions that create a novel site, but also introduce a *Nar*I site. The resulting 1,170 basepair PCR fragment was then introduced into YFM5.2(*Nar*I[+]*Sph*I[-]).

The 5' portion of the DEN-2 clone including the prM and amino terminal portion of the E protein was engineered into the YF5'3'IV plasmid using a chimeric PCR primer. The chimeric primer, incorporating the 3' end of negative-sense YF C protein and 5' end of DEN-2 prM protein, was used with a positive-sense primer flanking the SP6 promoter of the YF5'3'IV plasmid to generate a 771 basepair PCR product with a 20 base extension representing DEN-2 prM sequence. This PCR product was then used to prime the DEN-2 plasmid in conjunction with a 3' primer representing DEN-2 sequence 1,501-1,522 and flanking the *Sph*I, to generate an 1,800 basepair final PCR product including the YF sequence from the *Not*I site through the SP6 promoter, YF 5' untranslated region, and YF C protein, contiguous with the DEN-2 prM-E1522 sequence. The PCR product was ligated into YF5'3'IV using *Not*I and *Sph*I sites to yield the YF5'3'IV/DEN(prM-E) plasmid.

### Construction of Chimeric Templates for Other Flaviviruses

Procedures for generating full-length cDNA templates encoding chimeric YF/MVE, YF/SLE, YF/WN, YF/TBE viruses are similar to those described above for the YF/DEN-2 system. Table 6 illustrates the features of the strategy for generating YF17D-based chimeric viruses. The unique restriction sites used for *in vitro* ligation, and the chimeric primers for engineering the C/prM and E/NSI junctions are also shown. Sources of cDNA for these heterologous flaviviruses are readily available (MVE: Dalgarno et al., J. Mol. Biol. 187:309-323, 1986; SLE: Trent et al., Virology 156:293-304, 1987; TBE: Mandl et al., Virology 166:197-205, 1988; Dengue 1: Mason et al., Virology 161:262-267, 1987; Dengue 2: Deubel et al., Virology 155:365-377, 1986; Dengue 3: Hahn et al., Virology 162:167-180, 1988; Dengue 4: Zhao et al., Virology 155:77-88, 1986). Bray et al. (1991), Proc. Natl. Acad. Sci. 88:10342-10346 describes the construction of intertypic chimeric dengue viruses by substitution of structural protein genes. Furthermore, a recombinant DNA construct containing nucleic acid derived from at least two flaviviruses is disclosed in WO 93/06214. In this construct no more than two structural proteins from tick-borne encephalitis virus (TBEV) are included.

The document Venugopal & Gould (1994), Vaccine 12:966-975, reviews flavivirus vaccines. Pletnev et al. (1992), Proc. Natl. Acad. Sci. 89:10532-10536 describes the construction and characterization of chimeric tick-borne encephalitis/dengue type 4 viruses.

Full-length chimeric TBEV/DEN4 cDNAs were constructed by substituting TBEV genes coding for proteins such as capsid (C), pre-membrane (pre-M), envelope (E) or non-structural protein NS1 for the corresponding DEN4 sequences.

An alternative approach to engineering additional chimeric viruses is to create the C/prM junction by blunt end ligation of PCR-derived restriction fragments having ends that meet at this junction and 5' and 3' termini that flank appropriate restriction sites for introduction into YF5'3'IV or an intermediate plasmid such as pBS-KS(+). The option to use a chimeric oligonucleotide or blunt-end ligation will vary, depending on the availability of unique restriction sites within the envelope protein coding region of the virus in question.

### Construction of YF Viruses Encoding HCV Antigens

Because the structural proteins E1 and E2 of HCV are not homologous to the structural proteins of the flaviviruses described above, the strategy for expression of these proteins involves insertion within a nonessential region of the genome, such that all of these proteins are then co-expressed with yellow fever proteins during viral replication in infected cells. The region to be targeted for insertion of the proteins is the N terminal portion of the NS1 protein, since the entire NS1 protein is not required for viral replication. Because of the potential problems with stability of the YF genome in the presence of heterologous sequence exceeding the normal size of the genome (approximately 10,000 nucleotides), the detection strategy described below can be used. In addition, deletion of NS1 may be advantageous in the chimeric YF/Flavivirus systems described above, because partial deletion of this protein may abrogate the immunity to YF associated with antibodies against NS1, and thus avoid problems with vector immunity if more than one chimeric vaccine was to be needed in a given recipient, or if a YF vaccine had been previously given or needed at a future point.

The strategy involves creating a series of in-frame deletions within the NS1 coding region of the YFM5.2 plasmid, in conjunction with engineering a translational termination codon at the end of E, and a series of two IRESs (internal ribosome entry sites). One IRES is immediately downstream of the termination codon and allows for expression of an open reading frame within the region between E and NS1. The second IRES initiates translation from truncated NS1 proteins, providing expression of the remainder of the YF nonstructural polyprotein. These derivatives are tested for recovery of infectious virus and the construct with the largest deletion is used for insertion of foreign sequences (*e*.*g*., HCV proteins) in the first IRES. This particular construct can also serve as a basis for determining whether deletion of NS1 will affect vector-specific immunity in the context of YF/Flavivirus chimeric viruses expressing prM-E, as described above.

The insertion of nucleotides encoding E1, E2, and/or E1 plus E2 HCV proteins is limited by the size of the deletion tolerated in the NS1 protein. Because of this, truncated HCV proteins can be used to enhance stability within the modified YF clone. The HCV proteins are engineered with an N-terminal signal sequence immediately following the IRES and a termination codon at the C terminus. This construction will direct the HCV proteins into the endoplasmic reticulum for secretion from the cell. The strategy for this construction is shown schematically in Fig. 6. Plasmids encoding HCV proteins of genotype I can be used for these constructions, for example, HCV plasmids obtained from Dr. Charles Rice at Washington University (Grakoui *et* *al*., J. Virology 67:1385-1395, 1993), who has expressed this region of the virus in processing systems and within a replication-complement full-length HCV clone.

### PrM cleavage deletion mutants as attenuating vaccine candidates for flaviviruses

Additional chimeric viruses included in the invention contain mutations that prevent prM cleavage, such as mutations in the prM cleavage site. For example, the prM cleavage site in flavivirus infectious clones of interest, such as dengue, TBE, SLE, and others can be mutated by site-directed mutagenesis. Any or all of the amino acids in the cleavage site, as set forth above, can be deleted or substituted. A nucleic acid fragment containing the mutated prM-E genes can then be inserted into a yellow fever virus vector using the methods described above. The prM deletion can be used with or without other attenuating mutations, for example, mutations in the E protein, to be inserted into the yellow fever virus. These mutants have advantages over single substitution mutants as vaccine candidates, because it is almost impossible to revert the deleted sequence and restore virulence.

The following chimeric flaviviruses of the invention were deposited with the American Type Culture Collection (ATCC) in Rockville, Maryland, U.S.A. under the terms of the Budapest Treaty and granted a deposit date of January 6, 1998: Chimeric Yellow Fever 17D/Dengue Type 2 Virus (YF/DEN-2; ATCC accession number ATCC VR-2593) and Chimeric Yellow Fever 17D/Japanese Encephalitis SA₁₄-14-2 Virus (YF/JE A1.3; ATCC accession number ATCC VR-2594).

**Table 1**

| Characterization of YF/JE chimeras | | | | | |
|---|---|---|---|---|---|
| Clone | Yield (µg) | Infectivity plaques/100ng LLC-MK2 | PBS log titer VERO | RNAse log titer VERO | DNAse log titer VERO |
| YF5.21v | 5.5 | 15 | 7.2 | 0 | 7 |
| YF/JE-S | 7.6 | 50 | 6.2 | 0 | 6.2 |
| YF/JE-N | 7 | 60 | 5 | 0 | 5.4 |

**Table 2**

| Sequence comparison of JE strains and YF/JE chimeras | | | | | | | | |
|---|---|---|---|---|---|---|---|---|
| Virus | E 107 | E 138 | E 176 | E 177 | E 227 | E 243 | E 244 | E 279 |
| JE SA14-14-2 | F | K | V | T | S | K | G | M |
| YF/JE SA14-14-2 | F | K | V | A | S | E | G | M |
| YF/JE NAK | L E | | I | T | P | E | E | K |
| JE NAK | L E | | I | T | P | E | E | K |
| JE SA14 | L E | | I | T | S | E | G | K |

**Table 3**

| Plaque reduction neutralization titers on YF/JE chimeras | | | | | |
|---|---|---|---|---|---|
| Virus | non-immune ascitic fluid | YF ascitic fluid | JE ascitic fluid | non-immune IgG | YF IgG |
| YF5.2iv | <1.3 | 3.7 | <1.3 | <2.2 | >4.3 |
| JE SA14-14-2 | <1.3 | <1.3 | 3.4 | <2.2 | <2.2 |
| YF/JE SA 14-14-2 | <1.3 | <1.3 | 3.1 | <2.2 | <1.9 |
| YF/JE Nakayama | <1.3 | <1.3 | 3.4 | <2.2 | <2.2 |

**Table 4**

| Neurovirulence of YF/JE SA14-14-2 Chimera 3 week old male ICR mice | | | |
|---|---|---|---|
| | log dose I.C. | % Mortality | |
| YF5.2iv | 4 | 100 | (7/7) |
| YF/JE SA 14-14-2 | 4 | 0 | (0/7) |
| YF/JE SA 14-14-2 | 5 | 0 | (0/7) |
| YF/JE SA 14-14-2 | 6 | 0 | (0/8) |

**Table 5**

| Neuroinvasiveness of YE/JE Chimeras 3 week old male ICR mice | | | |
|---|---|---|---|
| | log dose (intraperitoneal) | % mortality | |
| YF/JE Nakayama | 4 | 0 | (0/5) |
| YF/JE Nakayama | 5 | 0 | (0/4) |
| YF/JE Nakayama | 6 | 0 | (0/4) |
| YF/JE SA14-14-2 | 4 | 0 | (0/5) |
| YF/JE SA 14-14-2 | 5 | 0 | (0/4) |
| YF/TE SA 14-14-2 | 6 | 0 | (0/4) |

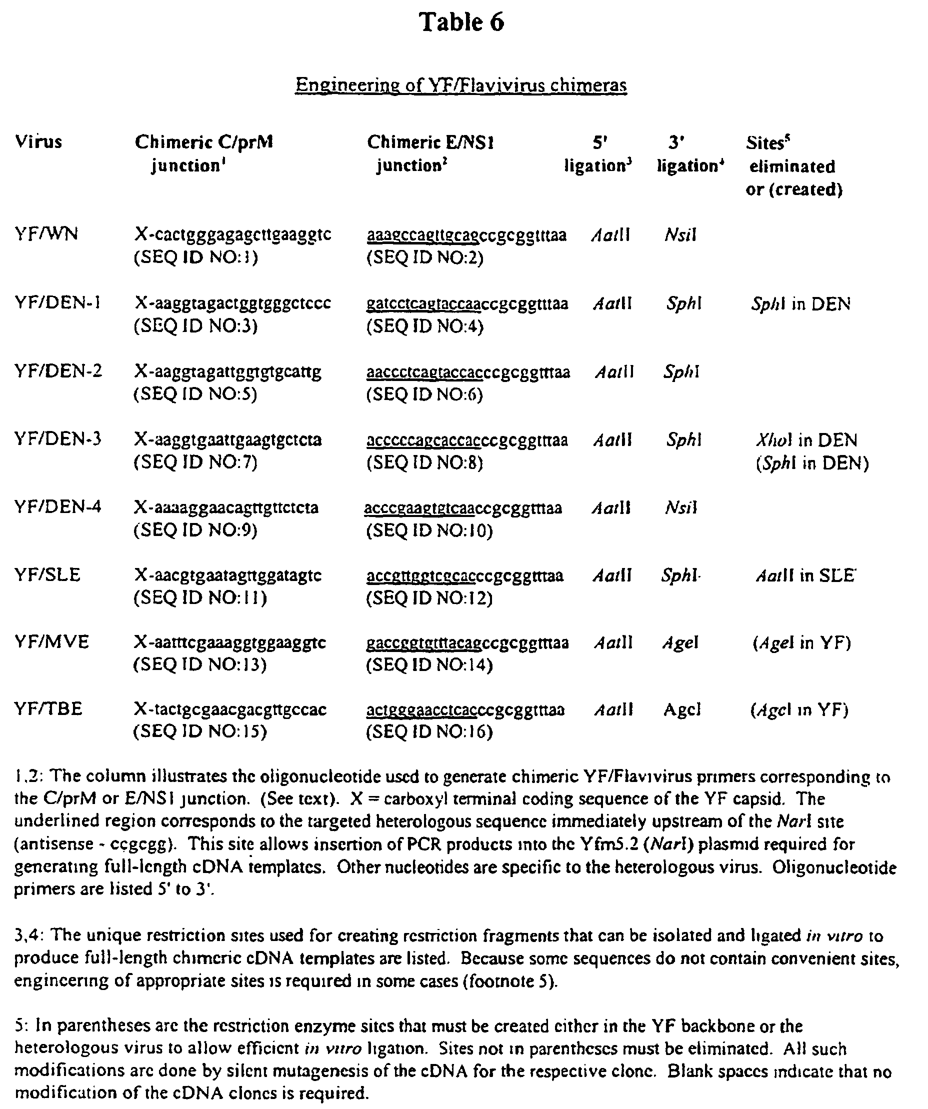

### Other Embodiments

Other embodiments are within the following claims. For example, the prM-E protein genes of other flaviviruses of medical importance can be inserted into the yellow fever vaccine virus backbone to produce vaccines against other medically important flaviviruses (see, *e*.*g*., Monath *et al*., "Flaviviruses," In *Virology*, Fields (ed.), Raven-Lippincott, New York, 1995, Volume 1, 961-1034).
Examples of additional flaviviruses from which genes to be inserted into the chimeric vectors of the invention can be obtained include, *e.g.*, Kunjin, Central European Encephalitis, Russian Spring-Summer Encephalitis, Powassan, Kyasanur Forest Disease, and Omsk Hemorrhagic Fever viruses. In addition, genes from even more distantly related viruses can be inserted into the yellow fever vaccine virus to construct novel vaccines.

### Vaccine Production and Use

The vaccines of the invention are administered in amounts, and by using methods, that can readily be determined by persons of ordinary skill in this art. The vaccines can be administered and formulated, for example, in the same manner as the yellow fever 17D vaccine, *e*.*g*., as a clarified suspension of infected chicken embryo tissue, or a fluid harvested from cell cultures infected with the chimeric yellow fever virus. Thus, the live, attenuated chimeric virus is formulated as a sterile aqueous solution containing between 100 and 1,000,000 infectious units (*e.g*., plaque-forming units or tissue culture infectious doses) in a dose volume of 0.1 to 1.0 ml, to be administered by, for example, intramuscular, subcutaneous, or intradermal routes. In addition, because flaviviruses may be capable of infecting the human host *via* the mucosal routes, such as the oral route (Gresikova *et al*., "Tick-borne Encephalitis," In *The Arboviruses. Ecology and Epidemiology*, Monath (ed.), CRC Press, Boca Raton, Florida, 1988, Volume IV, 177-203), the vaccine virus can be administered by a mucosal route to achieve a protective immune response. The vaccine can be administered as a primary prophylactic agent in adults or children at risk of flavivirus infection. The vaccines can also be used as secondary agents for treating flavivirus-infected patients by stimulating an immune response against the flavivirus.

It may be desirable to use the yellow fever vaccine vector system for immunizing a host against one virus (for example, Japanese Encephalitis virus) and to later reimmunize the same individual against a second or third virus using a different chimeric construct. A significant advantage of the chimeric yellow fever system is that the vector will not elicit strong immunity to itself. Nor will prior immunity to yellow fever virus preclude the use of the chimeric vaccine as a vector for heterologous gene expression. These advantages are due to the removal of the portion of the yellow fever vaccine E gene that encodes neutralizing (protective) antigens to yellow fever, and replacement with another, heterologous gene that does not provide cross-protection against yellow fever. Although YF17D virus nonstructural proteins may play a role in protection, for example, by eliciting antibodies against NS1, which is involved in complement-dependent antibody mediated lysis of infected cells (Schlesinger *et al*., J. Immunology 135:2805-2809, 1985), or by inducing cytotoxic T cell responses to NS3 or other proteins of the virus, it is unlikely that these responses will abrogate the ability of a live virus vaccine to stimulate neutralizing antibodies. This is supported by the facts that (1) individuals who have been previously infected with JE virus respond to vaccination with YF17D similarly to persons without previous JE infection, and (2) individuals who have previously received the YF17D vaccine respond to revaccination with a rise in neutralizing antibody titers (Sweet *et al*., Am. J. Trop. Med. Hyg. 11:562-569, 1962). Thus, the chimeric vector can be used in populations that are immune to yellow fever because of prior natural infection or vaccination, and can be used repeatedly, or to immunize simultaneously or sequentially with several different constructs, including yellow fever chimeras with inserts from, for example, Japanese Encephalitis, St. Louis Encephalitis, or West Nile viruses.

For vaccine applications, adjuvants that are known to those skilled in the art can be used. Adjuvants that can be used to enhance the immunogenicity of the chimeric vaccines include, for example, liposomal formulations, synthetic adjuvants, such as saponins (*e.g*., QS21), muramyl dipeptide, monophosphoryl lipid A, or polyphosphazine. Although these adjuvants are typically used to enhance immune responses to inactivated vaccines, they can also be used with live vaccines. In the case of a chimeric vaccine delivered via a mucosal route, for example, orally, mucosal adjuvants such as the heat-labile toxin of *E. coli* (LT) or mutant derivations of LT are useful adjuvants. In addition, genes encoding cytokines that have adjuvant activities can be inserted into the yellow fever vectors. Thus, genes encoding cytokines, such as GM-CSF, IL-2, IL-12, IL-13, or IL-5, can be inserted together with heterologous flavivirus genes to produce a vaccine that results in enhanced immune responses, or to modulate immunity directed more specifically towards cellular, humoral, or mucosal responses. In addition to vaccine applications, as one skilled in the art can readily understand, the vectors of the invention can be used in gene therapy methods to introduce therapeutic gene products into a patient's cells. In these methods, genes encoding therapeutic gene products are inserted into the vectors, for example, in place of the gene encoding the prM-E protein.

An additional advantage of the yellow fever vector system is that flaviviruses replicate in the cytoplasm of cells, so that the virus replication strategy does not involve integration of the viral genome into the host cell (Chambers *et al.* (1990) "Flavivirus Genome Organization, Expression, and Replication," In *Annual Review of Microbiology* 44:649-688), providing an important safety measure.

## Claims

1. A chimeric live, infectious, attenuated virus, comprising:
a yellow fever virus in which the nucleotide sequence encoding a prM-E protein is either deleted, truncated, or mutated so that functional yellow fever virus prM-E protein is not expressed, and
integrated into the genome of said yellow fever virus, a nucleotide sequence encoding a prM-E protein of a second, different flavivirus, so that said prM-E protein of said second flavivirus is expressed, wherein the signal sequence at the C/prM junction of said yellow fever virus is maintained.

2. The chimeric virus of claim 1, wherein said second flavivirus is a Japanese Encephalitis (JE) virus.

3. The chimeric virus of claim 1, wherein said second flavivirus is a Dengue virus selected from the group consisting of Dengue types 1-4.

4. The chimeric virus of claim 1, wherein said second flavivirus is selected from the group consisting of a Murray Valley Encephalitis virus, a St. Louis Encephalitis virus, a West Nile virus, a Tick-borne Encephalitis virus, a Hepatitis C virus, a Kunjin virus, a Central European Encephalitis virus, a Russian Spring-Summer Encephalitis virus, a Powassan virus, a Kyasanur Forest Disease virus, and an Omsk Hemorrhagic Fever virus.

5. The chimeric virus of claim 1, wherein the nucleotide sequence encoding the prM-E protein of said second, different flavivirus replaces the nucleotide sequence encoding the prM-E protein of said yellow fever virus.

6. The chimeric virus of claim 1, wherein said nucleotide sequence encoding said prM-E protein of said second, different flavivirus comprises a mutation that prevents prM cleavage to produce M protein.

7. The chimeric virus of claim 1, wherein the signal sequences at the C/prM and E/NS 1 junctions ofsaid yellow fever virus are maintained in construction of said chimeric flavivirus.

8. Use of a chimeric live, infectious, attenuated virus in the preparation of a medicament for preventing or treating flavivirus infection in a patient, wherein the chimeric, live, infectious attenuated virus comprises
a yellow fever virus in which the nucleotide sequence encoding a prM-E protein is either deleted, truncated, or mutated so that functional yellow fever virus prM-E protein is not expressed, and
integrated into the genome of said yellow fever virus, a nucleotide sequence encoding a prM-E protein of a second, different flavivirus, so that said prM-E protein of said second flavivirus is expressed, wherein the signal sequence at the C/prM junction of said yellow fever virus is maintained.

9. The use of claim 8, wherein said second flavivirus is a Japanese Encephalitis (JE) virus.

10. The use of claim 8, wherein said second flavivirus is a Dengue virus selected from the group consisting of Dengue types 1-4.

11. The use of claim 8, wherein said second flavivirus is selected from the group consisting of a Murray Valley Encephalitis virus, a St. Louis Encephalitis virus, a West Nile virus, a Tick-borne Encephalitis virus, a Hepatitis C virus, a Kunjin virus, a Central European Encephalitis virus, a Russian Spring-Summer Encephalitis virus, a Powassan virus, a Kyasanur Forest Disease virus, and an Omsk Hemorrhagic Fever virus.

12. The use of claim 8, wherein the nucleotide sequence encoding the prM-E protein of said second, different flavivirus replaces the nucleotide sequence encoding the prM-E protein of said yellow fever virus.

13. The use of claim 8, wherein said nucleotide sequence encoding said prM-E protein of said second, different flavivirus comprises a mutation that prevents prM cleavage to produce M protein.

14. The use of claim 8, wherein the signal sequences at the C/prM and E/NS 1 junctions of said yellow fever virus are maintained in construction of said chimeric flavivirus.

15. A nucleic acid molecule encoding a chimeric live, infectious, attenuated virus, said virus comprising:
a yellow fever virus in which the nucleotide sequence encoding a prM-E protein is either deleted, truncated, or mutated so that functional yellow fever virus prM-E protein is not expressed, and
integrated into the genome of said yellow fever virus, a nucleotide sequence encoding a prM-E protein ofa second, different flavivirus, so that said prM-E protein of said second flavivirus is expressed, wherein the signal sequence at the C/prM junction of said yellow fever virus is maintained.

16. The nucleic acid molecule of claim 15, wherein said second flavivirus is a Japanese Encephalitis (JE) virus.

17. The nucleic acid molecule of claim 15, wherein said second flavivirus is a Dengue virus selected from the group consisting of Dengue types 1-4.

18. The nucleic molecule of claim 15, wherein said second flavivirus is selected from the group consisting of a Murray Valley Encephalitis virus, a St. Louis Encephalitis virus, a West Nile virus, a Tick-borne Encephalitis virus, a Hepatitis C virus, a Kunjin virus, a Central European Encephalitis virus, a Russian Spring-Summer Encephalitis virus, a Powassan virus, a Kyasanur Forest Disease virus, and an Omsk Hemorrhagic Fever virus.

19. The nucleic acid molecule of claim 15, wherein the nucleotide sequence encoding the prM-E protein of said second, different flavivirus replaces the nucleotide sequence encoding the prM-E protein of said yellow fever virus.

20. The nucleic acid molecule of claim 15, wherein said nucleotide sequence encoding said prM-E protein of said second, different flavivirus comprises a mutation that prevents prM cleavage to produce M protein.

21. The nucleic acid molecule of claim 15, wherein the signal sequences at the C/prM and E/NS 1 junctions of said yellow fever virus are maintained in construction of said chimeric flavivirus.

## Patentansprüche

1. Ein chimärer lebender infektiöser attenuierter Virus, enthaltend:
einen Gelbfieber-Virus, in welchem die Nukleotidsequenz, die ein prM-E Protein kodiert, entweder deletiert, verkürzt oder mutiert ist, so dass funktionales Gelbfieber-Virus prM-E Protein nicht exprimiert wird, und
integriert in das Genom des Gelbfieber-Virus eine Nukleotidsequenz, die ein prM-E Protein eines zweiten verschiedenen Flavivirus kodiert, so dass das prM-E Protein des zweiten Flavivirus exprimiert wird, wobei die Signalsequenz an dem C/prM Übergang des Gelbfieber-Virus beibehalten wird.

2. Der chimäre Virus von Anspruch 1, wobei das zweite Flavivirus ein Japanischer-Enzephalitis (JE) Virus ist.

3. Der chimäre Virus von Anspruch 1, wobei das zweite Flavivirus ein Dengue-Virus ist ausgewählt aus der Gruppe bestehend aus Dengue Typen 1-4.

4. Der chimäre Virus von Anspruch 1, wobei das zweite Flavivirus ausgewählt ist aus der Gruppe bestehend aus einem Murray-Tal-Enzephalitis-Virus, einem St. Louis-Enzephalitis-Virus, einem West-Nil-Virus, einem Durch-Zecke-übertragenen-Enzephalitis-Virus, einem Hepatitis-C-Virus, einem Knunjin-Virus, einem Zentraleuropäischen-Enzephalitis-Virus, einem Russsischen-Frühling-Sommer-Enzephalitis-Virus, einem Powassan-Virus, einem Kyasanur-Wald-Krankheits-Virus und einem Omsk-Hämorrhagischen-Fiebervirus.

5. Der chimäre Virus von Anspruch 1, wobei die Nukleotidsequenz, die das prM-E Protein des zweiten verschiedenen Flavivirus kodiert, die Nukleotidsequenz ersetzt, die das prM-E Protein des Gelbfieber-Virus kodiert.

6. Der chimäre Virus von Anspruch 1, wobei die Nukleotidsequenz, die das prM-E Protein des zweiten verschiedenen Flavivirus kodiert, eine Mutation beinhaltet, die die prM Spaltung zur Produktion von M Protein verhindert.

7. Das chimäre Virus von Anspruch 1, wobei die Signalsequenz an den C/prM und E/NS1 Verbindungsstellen dieses Gelbfieber-Virus bei der Konstruktion des chimären Flavivirus beibehalten werden.

8. Verwendung eines chimären lebenden infektiösen attenuierten Virus für die Herstellung eines Medikamentes zum Verhindern oder Behandeln einer Flavivirus Infektion in einem Patienten, wobei der chimäre lebende infektiöse attenuierte Virus enthält
einen Gelbfieber-Virus, in welchem die Nukleotidsequenz, die ein prM-E Protein kodiert, entweder deletiert, verkürzt, oder mutiert ist, so dass das funktionale Gelbfieber-Virus prM-E Protein nicht exprimiert wird, und
integriert in das Genom des Gelbfieber-Virus, eine Nukleotidsequenz, die ein prM-E Protein eines zweiten verschiedenen Flavivirus kodiert, so dass dieses prM-E Protein des zweiten Flavivirus exprimiert wird, wobei die Signalsequenz an dem C/prM Übergang dieses Gelbfieber-Virus beibehalten wird.

9. Die Verwendung von Anspruch 8, wobei das zweite Flavivirus ein Japanischer-Enzephalitis (JE) Virus ist.

10. Die Verwendung von Anspruch 8, wobei das zweite Flavivirus ein Dengue-Virus ist ausgewählt aus der Gruppe bestehend aus Dengue Typen 1-4.

11. Die Verwendung von Anspruch 8, wobei das zweite Flavivirus ausgewählt ist aus der Gruppe bestehend aus einem Murray-Tal-Enzephalitis-Virus, einem St. Louis-Enzephalitis-Virus, einem West-Nil-Virus, einem Durch-Zecke-übertragenen-Enzephalitis-Virus, einem Hepatitis-C-Virus, einem Knunjin-Virus, einem Zentraleuropäischen-Enzephalitis-Virus, einem Russsischen-Frühling-Sommer-Enzephalitis-Virus, einem Powassan-Virus, einem Kyasanur-Wald-Krankheits-Virus und einem Omsk-Hämorrhagischen-Fiebervirus.

12. Die Verwendung von Anspruch 8, wobei die Nukleotidsequenz, die das prM-E Protein des zweiten verschiedenen Flavivirus kodiert, die Nukleotidsequenz ersetzt, die das prM-E Protein des Gelbfieber-Virus kodiert.

13. Die Verwendung von Anspruch 8, wobei die Nukleinsäuresequenz, die das prM-E Protein des zweiten verschiedenen Flavivirus kodiert, eine Mutation enthält, die die prM Spaltung zur Produktion von M Protein verhindert.

14. Die Verwendung von Anspruch 8, wobei die Signalsequenz an den C/prM und E/NS1 Verbindungsstellen dieses Gelbfieber-Virus bei der Konstruktion des chimären Flavivirus beibehalten werden.

15. Ein Nukleinsäuremolekül, das einen chimären lebenden infektiösen attenuierten Virus kodiert, wobei das Virus enthält:
einen Gelbfieber-Virus, in welchem die Nukleotidsequenz, die ein prM-E Protein kodiert, entweder deletiert, verkürzt, oder mutiert ist, so dass das funktionale Gelbfieber-Virus prM-E Protein nicht exprimiert wird, und
integriert in das Genom des Gelbfieber-Virus, ein Nukleotidsequenz, die ein prM-E Protein eines zweiten verschiedenen Flavivirus kodiert, so dass das prM-E Protein des zweiten Flavivirus exprimiert wird, wobei die Signalsequenz an dem C/prM Übergang des Gelbfieber-Virus beibehalten wird.

16. Das Nukleinsäuremolekül von Anspruch 15, wobei das zweite Flavivirus ein Japanischer-Enzephalitis (JE) Virus ist.

17. Das Nukleinsäuremolekül von Anspruch 15, wobei das zweite Flavivirus ein Dengue-Virus ist ausgewählt aus der Gruppe bestehend aus Dengue Typen 1-4.

18. Das Nukleinsäuremolekül von Anspruch 15, wobei das zweite Flavivirus ausgewählt ist aus der Gruppe bestehend aus einem Murray-Tal-Enzephalitis-Virus, einem St. Louis-Enzephalitis-Virus, einem West-Nil-Virus, einem Durch-Zecke-übertragenem-Enzephalitis-Virus, einem Hepatitis-C-Virus, einem Knunjin-Virus, einem Zentraleuropäischen-Enzephalitis-Virus, einem Russsischen-Frühling-Sommer-Enzephalitis-Virus, einem Powassan-Virus, einem Kyasanur-Wald-Krankheits-Virus, und einem Omsk-Hämorrhagischen-Fibervirus.

19. Das Nukleinsäuremolekül von Anspruch 15, wobei die Nukleotidsequenz, die das prM-E Protein des zweiten verschiedenen Flavivirus kodiert, die Nukleotidsequenz ersetzt, die das prM-E Protein des Gelbfieber-Virus kodiert.

20. Das Nukleinsäuremolekül von Anspruch 15, wobei die Nukleinsäuresequenz, die das prM-E Protein des zweiten verschiedenen Flavivirus kodiert, eine Mutation enthält, die die prM Spaltung zur Produktion von M Protein verhindert.

21. Das Nukleinsäuremolekül von Anspruch 15, wobei die Signalsequenz an den C/prM und E/NS1 Verbindungsstellen des Gelbfieber-Virus bei der Konstruktion des chimären Flavivirus beibehalten werden.

## Revendications

1. Virus chimère vivant, infectieux, atténué, comprenant
un virus de la fièvre jaune dans lequel la séquence nucléotidique codant pour une protéine prM-E est délétée, tronquée ou mutée de telle sorte que la protéine prM-E fonctionnelle du virus de la fièvre jaune n'est pas exprimée, et
intégrée dans le génome du dit virus de la fièvre jaune, une séquence nucléotidique codant pour une protéine prM-E d'un deuxième flavivirus, différent, de telle sorte que ladite protéine prM-E du dit deuxième flavivirus est exprimée, tandis que la séquence signal à la jonction C/prM du dit virus de la fièvre jaune est maintenue.

2. Virus chimère selon la revendication 1, dans lequel ledit deuxième flavivirus est un virus de l'encéphalite japonaise (EJ).

3. Virus chimère selon la revendication 1, dans lequel ledit deuxième flavivirus est un virus de la Dengue choisi dans le groupe consistant en les types de Dengue 1-4.

4. Virus chimère selon la revendication 1, dans lequel ledit deuxième flavivirus est choisi dans le groupe consistant en un virus de l'encéphalite de la Murray Valley, un virus de l'encéphalite de Saint-Louis, un virus West Nile, un virus de l'encéphalite transmise par les tiques, un virus de l'hépatite C, un virus Kunjin, un virus de l'encéphalite d'Europe centrale, un virus de l'encéphalite verno-estivale russe, un virus Powassan, un virus de la forêt de Kyasanur, et un virus de la fièvre hémorragique d'Omsk.

5. Virus chimère selon la revendication 1, dans lequel la séquence nucléotidique codant pour la protéine prM-E du dit deuxième flavivirus, différent, remplace la séquence nucléotidique codant pour la protéine prM-E du dit virus de la fièvre jaune.

6. Virus chimère selon la revendication 1, dans lequel la séquence nucléotidique codant pour la protéine prM-E du dit deuxième flavivirus, différent, comprend une mutation qui empêche le clivage de prM pour produire la protéine M.

7. Virus chimère selon la revendication 1, dans lequel les séquences signal sur les jonctions C/prM et E/NS 1 du dit virus de la fièvre jaune sont maintenues dans la construction du dit flavivirus chimère.

8. Utilisation d'un virus chimère vivant, infectieux, atténué, dans la préparation d'un médicament pour la prévention ou le traitement d'une infection par flavivirus chez un patient, dans laquelle le virus chimère vivant, infectieux, atténué, comprend:
un virus de la fièvre jaune dans lequel la séquence nucléotidique codant pour une protéine prM-E est délétée, tronquée ou mutée de telle sorte que la protéine prM-E fonctionnelle du virus de la fièvre jaune n'est pas exprimée, et
intégrée dans le génome du dit virus de la fièvre jaune, une séquence nucléotidique codant pour une protéine prM-E d'un deuxième flavivirus, différent, de telle sorte que ladite protéine prM-E du dit deuxième flavivirus est exprimée, tandis que la séquence signal à la jonction C/prM du dit virus de la fièvre jaune est maintenue.

9. Utilisation selon la revendication 8, dans laquelle ledit deuxième flavivirus est un virus de l'encéphalite japonaise (EJ).

10. Utilisation selon la revendication 8, dans laquelle ledit deuxième flavivirus est un virus de la Dengue choisi dans le groupe consistant en les types de Dengue 1-4.

11. Utilisation selon la revendication 8, dans laquelle ledit deuxième flavivirus est choisi dans le groupe consistant en un virus de l'encéphalite de la Murray Valley, un virus de l'encéphalite de Saint-Louis, un virus West Nile, un virus de l'encéphalite transmise par les tiques, un virus de l'hépatite C, un virus Kunjin, un virus de l'encéphalite d'Europe centrale, un virus de l'encéphalite verno-estivale russe, un virus Powassan, un virus de la forêt de Kyasanur, et un virus de la fièvre hémorragique d'Omsk.

12. Utilisation selon la revendication 8, dans laquelle la séquence nucléotidique codant pour la protéine prM-E du dit deuxième flavivirus, différent, remplace la séquence nucléotidique codant pour la protéine prM-E du dit virus de la fièvre jaune.

13. Utilisation selon la revendication 8, dans laquelle ladite séquence nucléotidique codant pour ladite protéine prM-E du dit deuxième flavivirus, différent, comprend une mutation qui empêche le clivage de prM pour produire la protéine M.

14. Utilisation selon la revendication 8, dans laquelle les séquences signal sur les jonctions C/prM et E/NS 1 du dit virus de la fièvre jaune sont maintenues dans la construction du dit flavivirus chimère.

15. Molécule d'acide nucléique codant pour un virus chimère vivant, infectieux, atténué, ledit virus comprenant:
un virus de la fièvre jaune dans lequel la séquence nucléotidique codant pour une protéine prM-E est délétée, tronquée ou mutée de telle sorte que la protéine prM-E fonctionnelle du virus de la fièvre jaune n'est pas exprimée, et
intégrée dans le génome du dit virus de la fièvre jaune, une séquence nucléotidique codant pour une protéine prM-E d'un deuxième flavivirus, différent, de telle sorte que ladite protéine prM-E du dit deuxième flavivirus est exprimée, tandis que la séquence signal à la jonction C/prM du dit virus de la fièvre jaune est maintenue.

16. Molécule d'acide nucléique selon la revendication 15, dans laquelle ledit deuxième flavivirus est un virus de l'encéphalite japonaise (EJ).

17. Molécule d'acide nucléique selon la revendication 15, dans laquelle ledit deuxième flavivirus est un virus de la Dengue choisi dans le groupe consistant en les types de Dengue 1-4.

18. Molécule d'acide nucléique selon la revendication 15, dans laquelle ledit deuxième flavivirus est choisi dans le groupe consistant en un virus de l'encéphalite de la Murray Valley, un virus de l'encéphalite de Saint-Louis, un virus West Nile, un virus de l'encéphalite transmise par les tiques, un virus de l'hépatite C, un virus Kunjin, un virus de l'encéphalite d'Europe centrale, un virus de l'encéphalite verno-estivale russe, un virus Powassan, un virus de la forêt de Kyasanur, et un virus de la fièvre hémorragique d'Omsk.

19. Molécule d'acide nucléique selon la revendication 15, dans laquelle la séquence nucléotidique codant pour la protéine prM-E du dit deuxième flavivirus, différent, remplace la séquence nucléotidique codant pour la protéine prM-E du dit virus de la fièvre jaune.

20. Molécule d'acide nucléique selon la revendication 15, dans laquelle ladite séquence nucléotidique codant pour ladite protéine prM-E du dit deuxième flavivirus, différent, comprend une mutation qui empêche le clivage de prM pour produire la protéine M.

21. Molécule d'acide nucléique selon la revendication 15, dans laquelle les séquences signal sur les jonctions C/prM et E/NS 1 du dit virus de la fièvre jaune sont maintenues dans la construction du dit flavivirus chimère.


### SEQUENCE LISTING

(1) GENERAL INFORMATION
   (i) APPLICANT: OraVax, Inc., et al.
   (ii) TITLE OF THE INVENTION: CHIMERIC FLAVIVIRUS VACCINES
   (iii) NUMBER OF SEQUENCES: 16
   (iv) CORRESPONDENCE ADDRESS:
      (A) ADDRESSEE: Clark & Elbing LLP
      (B) STREET: 176 Federal Street
      (C) CITY: Boston
      (D) STATE: MA
      (E) COUNTRY: USA
      (F) ZIP: 02110
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ for Windows Version 2.0
   (vi) CURRENT APPLICATION DATA:
      (A) APPLICATION NUMBER:
      (B) FILING DATE: 02-MAR-98
      (C) CLASSIFICATION:
   (vii) PRIOR APPLICATION DATA:
      (A) APPLICATION NUMBER: 08/807,445
      (B) FILING DATE: 28-FEB-1997
   (viii) PRIOR APPLICATION DATA:
      (A) APPLICATION NUMBER: unknown
      (B) FILING DATE: 15-JAN-98
   (ix) ATTORNEY/AGENT INFORMATION:
      (A) NAME: Clark, Paul T
      (B) REGISTRATION NUMBER: 30,162
      (C) REFERENCE/DOCKET NUMBER: 06132/033W02
   (x) TELECOMMUNICATION INFORMATION:
      (A) TELEPHONE: 617-428-0200
      (B) TELEFAX: 617-428-7045
      (C) TELEX:
(2) INFORMATION FOR SEQ ID NO:1:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:1:
   CACTGGGAGA GCTTGAAGGT C 21
(2) INFORMATION FOR SEQ ID NO:2:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:2:
   AAAGCCAGTT GCAGCCGCGG TTTAA 25
(2) INFORMATION FOR SEQ ID NO:3:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:3:
   AAGGTAGACT GGTGGGCTCC C 21
(2) INFORMATION FOR SEQ ID NO:4:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
   GATCCTCAGT ACCAACCGCG GTTTAA 26
(2) INFORMATION FOR SEQ ID NO:5:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
   AAGGTAGATT GGTGTGCATT G 21
(2) INFORMATION FOR SEQ ID NO:6:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:6:
   AACCCTCAGT ACCACCCGCG GTTTAA 26
(2) INFORMATION FOR SEQ ID NO:7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:7:
   AAGGTGAATT GAAGTGCTCT A 21
(2) INFORMATION FOR SEQ ID NO:8:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 8:
   ACCCCCAGCA CCACCCGCGG TTTAA 25
(2) INFORMATION FOR SEQ ID NO:9:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
   AAAAGGAACA GTTGTTCTCT A 21
(2) INFORMATION FOR SEQ ID NO.:10:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:10:
   ACCCGAAGTG TCAACCGCGG TTTAA 25
(2) INFORMATION FOR SEQ ID NO:11:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:11:
   AACGTGAATA GTTGGATAGT C 21
(2) INFORMATION FOR SEQ ID NO:12:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:12:
   ACCGTTGGTC GCACCCGCGG TTTAA 25
(2) INFORMATION FOR SEQ ID NO:13:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:13:
   AATTTCGAAA GGTGGAAGGT C 21
(2) INFORMATION FOR SEQ ID NO:14:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:14:
   GACCGGTGTT TACAGCCGCG GTTTAA 26
(2) INFORMATION FOR SEQ ID NO:15:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:15:
   TACTGCGAAC GACGTTGCCA C 21
(2) INFORMATION FOR SEQ ID NO:16:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:16:
   ACTGGGAACC TCACCCGCGG TTTAA 25